# EUROPÄISCHE PATENTSCHRIFT

(11) **EP 2 793 815 B1**
(45) Veröffentlichungstag und Bekanntmachung des Hinweises auf die Patenterteilung: **11.10.2023**
(21) Anmeldenummer: 12806430.0
(22) Anmeldetag: 14.12.2012
(51) Int. Cl.: A61K 8/34, A61Q 5/00, A61K 8/40, A61Q 15/00, A61Q 17/00, A61K 31/08, A61K 31/16, A61K 9/00

(54) **VERWENDUNG VON DERMATOLOGISCHEN ZUBEREITUNG UMFASSEND EINE WIRKSTOFFKOMBINATIONEN AUS EINER ODER MEHREREN ALKYLETHERN DES GLYCERINS UND EINER ODER MEHREREN PHYSIOLOGISCH UNBEDENKLICHEN HYDROXAMSÄUREN**
USE OF DERMATOLOGICAL PREPARATION COMPRISING A COMBINATION OF ACTIVE INGREDIENTS OF ONE OR MORE ALKYL ETHERS OF GLYCEROL AND ONE OR MORE PHYSIOLOGICALLY SAFE HYDROXAMIC ACIDS
UTILISATION D'UNE PRÉPARATION DERMATOLOGIQUE COMPRENANT UNE ASSOCIATION DE PRINCIPES ACTIFS D'UN OU PLUSIEURS ALKYL ETHERS DE GLYCEROL ET D'UN OU PLUSIEURS ACIDES HYDROXAMIQUES PHYSIOLOGIQUEMENT SANS DANGER

(30) Priorität: 19.12.2011 DE 102011088936
(43) Veröffentlichungstag der Anmeldung: 29.10.2014
(73) Patentinhaber: Inolex GmbH, 10117 Berlin (DE)
(72) Erfinder: FIRYN, Andreas, 21493 Schwarzenbek (DE); GÖKSEL, Hülya, 50733 Köln (DE); KÖHLER, Manuela, 22147 Hamburg (DE); TRAUPE, Bernd, 24568 Kaltenkirchen (DE)
(74) Vertreter: J A Kemp LLP
(86) Internationale Anmeldenummer: PCT/EP2012/075564
(87) Internationale Veröffentlichungsnummer: WO 2013/092417

(56) Entgegenhaltungen:
- WO-A1-2009/070736
- US-A- 4 847 069

## Beschreibung

Die vorliegende Erfindung betrifft eine dermatologische Zubereitung zur Verwendung als antibakterielles, antimykotisches oder antivirales Mittel wie in den Ansprüchen beschrieben.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, einen dermatologischen Zubereitung zur Verwendung als antibakterielles, antimykotisches oder antivirales Mittel zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen eine dermatologische Zubereitung zur Verwendung gegen unreine Haut bzw. Propionibacterium acnes zu finden Die vorliegende Offenlegung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, dass die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Die vorliegende Offenbarung betrifft weiter kosmetische Desodorantien, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin betrifft diese Offenbarung kosmetische Desodorantien, die sich durch gute Hautverträglichkeit auszeichnen. Auf keinen Fall sollten die desodorierenden Wirkprinzipien sich auf der Haut anreichern.

Diese Offenbarung betrifft kosmetische Desodorantien, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Diese Offenbarung betrifft kosmetische Desodorantien, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκηζ = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophyten (z.B. Epidermophytie, Favus, Mikrosporie, Trichophytie), Hefemycosen (z.B. Pityriasis und andere Pityrosporum-bedingte Mykosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophyten befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ein weit verbreitetes Phänomen sind die sogenannten Kopfschuppen. Produzieren die Talgdrüsen eines Menschen zu viel Talg oder sind diese Talgdrüsen durch zu seltenes Haare waschen durch Talg verstopft, dann wird dem normalerweise harmlose Hefepilz Pitysporum ovale, heute bekannt als Malassezia Furfur, ein starkes Wachstum ermöglicht, was zum Seborrhöischen Ekzem mit Juckreiz und Schuppenbildung führt.

Eine Aufgabe der vorliegenden Erfindung war, die Nachteile des Standes der Technik zu beseitigen und eine dermatologische Zubereitung zur Verwendung als antibakterielles, antimykotisches oder antivirales Mittel zur Verfügung zu stellen, die die Bildung von Kopfschuppen zu unterbinden, wobei die physiologische Hautflora keine nennenswerte Einbußen erleidet.

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde. Ferner haben topisch verabreichte Antibiotika den Nachteil, dass sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und eine dermatologische Zubereitung zur Verwendung als antibakterielles, antimykotisches oder antivirales Mittel zur Verfügung zu stellen, wodurch Superinfektionen geheilt werden können, wobei die physiologische Hautflora keine nennenswerte Einbußen erleidet. Protozoen sind parasitisch lebende Einzeller mit klar abgegrenztem Zellkern, die sich ungeschlechtlich fortpflanzen (durch Zwei- oder Vierfachteilung sowie Knospung), oder aber geschlechtlich (Gameto-, Gamonto- und Autogamie). Die Nahrungsaufnahme aus der Umgebung erfolgt durch Permeation sowie durch Pino- oder Phagozytose. Die meisten Protozoen können neben vegetativen, meist beweglichen Zustandsformen (sogenannten Trophozoiten) unter ungünstigen Umständen auch Zysten als Dauerformen ausbilden,

Je nach Fortbewegungsart und -apparat werden Protozoen in vier verschiedene Gruppen unterteilt:
(a) Mastigophora (Flagellaten mit Geißeln)
(b) Sarcodina/Rhizopoda (amöboides Bewegungsmuster durch Plasmaausstülpungen)
(c) Sporozoa (schlängelndes oder gleitendes Bewegungsmuster)
(d) Ciliata/Ciliophora (Bewimperung oder Begeißelung)

Parasitisch lebende Protozoen werden in subtropischen und tropischen Gebieten häufig durch stechende und saugende Insekten, aber auch Schmutz- und Schmierinfektion sowie durch die Nahrungskette übertragen.

Einige medizinisch und dermatologisch relevante Protozoonosen sind: Trichomoniasis (verursacht von Trichomonas vaginalis), Lamblienruhr (verursacht durch Lamblia intestinalis), viszerale sowie kutane und Schleimhaut-Leishmaniose (verursacht beispielsweise durch Leishmania donovanii, L.tropica, L.brasiliensis, L-mexicana, L.diffusa oder L. pifanoi), Trypanosmiasis (verursacht durch verschiedene Trypanosoma-Arten), Amöbenruhr und Amöbiasis (verursacht beispielsweise durch verschiedene Entamoeba-Arten, Jodamoeba butschlii oder Naegleria fowleri), Kokzidose (durch Isospora belli) und Balantidenruhr (verursacht durch Balantidium coli).

Durch Protozoonosen hervorgerufene medizinische und dermatologische Phänomene beeinträchtigen, zum Teil erheblich, das menschliche Wohlbefinden. Es besteht daher bei den betroffenen Personen ein erheblicher Bedarf, diesem Zustande abzuhelfen. Eine Aufgabe der vorliegenden Erfindung war es also, gegen Protozoen wirksame Wirkprinzipien zu finden.

Im Gegensatze zu den prokaryotischen und eukaryotischen zellulären Organismen sind Viren [Virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen sind jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Offenlegung war also, diesem Übelstande abzuhelfen, also Substanzen zu finden, welche wirksam einen ein- oder mehrzelligen Organismus vor schädlichen Folgen einer Virusinfektion, sei es prophylaktisch oder therapeutisch, zu schützen.

Kosmetische Zubereitungen müssen darüber hinaus langzeitstabil gegen mikrobielle Kontamination formuliert werden.

Die mikrobielle Stabilität wird bislang durch den Zusatz an Konservierungsmitteln gelöst. Bekannte Konservierungsmittel sind zum Beispiel die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.

Zubereitungen ohne oder möglichst geringer Menge an Konservierungsmittel bereit zu stellen ist jedoch ein Wunsch der Konsumenten.

Eine Aufgabe der vorliegenden Offenlegung war also, diesem Übelstande abzuhelfen, also Substanzen zu finden, welche wirksam gegen gram positive, gram negative, Hefen und Pilze wirken und somit ein Produkt auch über einen langen Zeitraum von mindestens 3 Jahren vor mikrobielle Kontamination zu schützen und dabei möglichst geringe Mengen an antimikrobiell wirkenden Substanzen zu verwenden.

Es wurde gefunden, und darin liegt die Lösung all dieser Aufgaben, daß eine dermatologische Zubereitung zur Verwendung als antibakterielles, antimykotisches oder antivirales Mittel, wobei die dermatologische Zubereitung eine Wirkstoffkombination umfasst, wobei die Wirkstoffkombination umfasst
a) eine oder mehrere physiologisch unbedenkliche Hydroxamsäuren
b) einen oder mehrere Alkylether des Glycerins der allgemeinen Strukturformel 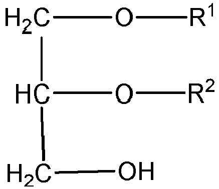
wobei R¹ und R² unabhängig voneinander darstellen:
verzweigte oder unverzweigte Alkylreste mit 1 - 18 Kohlenstoffatomen, und
wobei der eine oder mehrere Alkylether des Glycerins in einer Konzentration von 0,001 bis 5,0 Gew.-% der Zubereitung vorliegen.
bzw. die Verwendung solcher Wirkstoffkombinationen als gegen Bakterien, Mycota und, insbesondere als Wirkprinzip gegen Kopfschuppen, Viren, den Nachteilen des Standes der Technik abhelfen.

Hydroxamsäuren sind eine Klasse chemischer Verbindungen, die als funktionelle Gruppe die Gruppierung -CO-NHOH enthalten. Ein Beispiel ist die Octanohydroxamsäure, auch Caprylohydroxamsäure genannt, welche durch folgende Struktur gekennzeichnet ist.

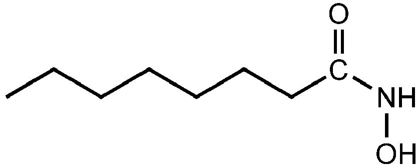

Sie ist ein bekannter Wirkstoff zur Konservierung kosmetischer Zubereitungen (z.B. WO 2009/070736 A1)

Erfindungsgemäß bevorzugte Alkylether des Glycerins werden gewählt aus der Gruppe der verzweigtkettigen Alkylether mit 4 bis 14 C-Atomen. Insbesondere vorteilhaft wird das Ethylhexylglycerin gewählt.

Es ist erfindungsgemäß vorteilhaft, die Gewichtsverhältnisse von Hydroxamsäure, insbesondere Octanohydroxamsäure, einerseits zu der Gesamtmenge an einem oder mehreren Alkylethern des Glycerins andererseits aus dem Bereich von 1 zu 10 bis 10 zu 1, bevorzugt von 1 zu 5 bis 5 zu 1, insbesondere bevorzugt von 1 zu 2 bis 2 zu 1 zu wählen.

Vorteilhaft werden die erfindungsgemäßen Wirkstoffkombinationen in kosmetischen oder dermatologischen Zubereitungen eingesetzt.

Erfindungsgemäße Zubereitungen enthalten vorteilhaft 0,001 bis 5,0 Gew.-% Hydroxamsäure, insbesondere Octanohydroxamsäure, bevorzugt 0,01 bis 2,0 Gew.-%, bezogen auf das Gesamtgewicht der Zubereitungen.

Erfindungsgemäße Zubereitungen enthalten 0,001 bis 5,0 Gew.-% an einem oder mehreren Alkylethern des Glycerins bevorzugt 0,01 bis 2,0 Gew.-%, jeweils bezogen auf das Gesamtgewicht der Zubereitungen.

Die erfindungsgemäßen Zubereitungen stellen in jeglicher Hinsicht überaus befriedigende Präparate dar.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Es hat sich in erstaunlicher Weise herausgestellt, dass die erfindungsgemäßen Wirkstoffkombinationen das Wachstum von Staphylococcus. Aureus verhindern, und dies in synergistischer Weise, also überadditiv in Bezug auf die Einzelkomponenten.

Es hat sich in erstaunlicher Weise herausgestellt, daß die erfindungsgemäß verwendeten Wirkstoffe das Wachstum von grampositiven und gramnegativen Bakterien, Mycobionten.

Die erfindungsgemäß verwendeten Wirkstoffe eignen sich darüber hinaus gut für die Verwendung als desodorierender Wirkstoff in kosmetischen Desodorantien sowie gegen unreine Haut, leichte Formen der Akne bzw. Propionibakterium acnes.

Die erfindungsgemäßen Wirkstoffe haben sich ebenfalls als besonders wirkungsvoll gegen Streptokokken erwiesen..

Schließlich hat sich herausgestellt, daß die erfindungsgemäß verwendeten Wirkstoffe den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden

Erfindungsgemäß sind somit auch ein Verfahren zur Bekämpfung von Mycobionten, dadurch gekennzeichnet, dass die erfindungsgemäß verwendeten Wirkstoffe gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, dass diesen organischen Produkten die erfindungsgemäß verwendeten Wirkstoffe in wirksamer Menge zugegeben werden.

Die erfindungsgemäßen Wirkstoffkombinationen sind ausgezeichnet wirksam gegen Mycobionten, insbesondere in deren Auswirkungsform der Dermatomycosen. Dabei sind die erfindungsgemäßen Wirkstoffkombinationen insbesondere befähigt, das Wachstum von Hefen, insbesondere der Pityrosporum-Arten, namentlich Pityrosporum ovale, zu verhindern.

Es hat sich ferner in überraschender Weise herausgestellt, dass die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Erfindungsgemäß sind somit auch ein Verfahren zur Bekämpfung seborrhoischer Erscheinungen, insbesondere Kopfschuppen, sowie die Prophylaxe seborrhoischer Erscheinungen, insbesondere Kopfschuppen.

Schließlich hat sich herausgestellt, dass die erfindungsgemäßen Wirkstoffkombinationen den Verderb organischer Substanz, insbesondere kosmetischer und dermatologischer Zubereitungen, durch den Befall mit Mycobionten verhindern können, wenn sie diesen Zubereitungen zugesetzt werden.

Erfindungsgemäß sind somit auch ein Verfahren zur Bekämpfung von Mycobionten, dadurch gekennzeichnet, dass die erfindungsgemäßen Wirkstoffkombinationen, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, dass diesen organischen Produkten erfindungsgemäße Wirkstoffkombinationen in wirksamer Menge zugegeben werden.

Ferner war erstaunlich, dass die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter, UVB und/oder mindestens einen Breitbandfilter und/oder mindestens ein anorganisches Pigment.
Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Puder, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Ganz besonders vorteilhaft enthalten die erfindungsgemäßen Zubereitungen

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB und UVA-Bereich absorbieren, wobei die Gesamtmenge der Filtersubstanzen z.B. 0,1 Gew.-% bis 50 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, Wirkstoffkombinationen gemäß der Erfindung, und Hilfsmittel, wie sie üblicherweise dafür verwendet werden.

Die folgenden Beispiele sollen die Verkörperungen der vorliegenden Erfindungen verdeutlichen. Die Angaben beziehen sich stets auf Gewichts-%, sofern nicht andere Angaben gemacht werden.

### O/W Emulsion:

| Beispiel Nr. | 1 | 2 | 3 | 4 | 5 |
|---|---|---|---|---|---|
| Panthenol | 1,5 | 0 | 0 | 0,7 | 0 |
| Octanohydroxamsäure | 0,25 | 0,2 | 0,3 | 0,1 | 0 |
| Glycerylcaprylat | 0,5 | 0 | 0,3 | 0 | 0,5 |
| Ethylhexylglycerin | 0 | 0,3 | 0 | 0,5 | 0,5 |
| Paraffinum Liquidum | 0 | 3 | 3 | 0 | 0 |
| Isopropyl Palmitat | 1 | 0 | 0 | 0 | 0 |
| Caprylic/Capric Triglycerid | 0 | 3 | 3 | 0 | 1,00 |
| Cetearylalkohol | 0 | 0 | 0 | 2,5 | 4,00 |
| Cetylalkohol | 0 | 3 | 3 | 0 | 0 |
| Paraffinum Liquidum | 0 | 3 | 3 | 0 | 0 |
| Dimethicon | 0 | 3 | 0 | 0 | 0,50 |
| Cyclomethicon D5 | 0 | 0 | 3 | 0 | 0 |
| C12-15 Alkylbenzoat | 0 | 0 | 0 | 0 | 2,00 |
| Butyrospermum Parkii Butter | 0 | 0,5 | 0,5 | 0 | 0 |
| Dicaprylylether | 0 | 0 | 0 | 10 | 0 |
| Dicaprylylcarbonat | 2 | 0 | 0 | 0 | 0 |
| Glyceryl Stearate | 0 | 1,2 | 1,2 | 2,4 | 2,4 |
| Tapiocastärke aq. | 1 | 0 | 0 | 0 | 0 |
| Glycerin | 5 | 8 | 10 | 8 | 8 |
| Zitronensäure | 0,0050 | 0 | 0 | 0 | 0 |
| Natronlauge aq 45% | 0,35 | 0,01 | 0,01 | 0,25 | 1 |
| Polyglyceryl-10 Myristat | 0 | 1,8 | 0 | 0 | 0 |
| Polyglyceryl-10 Stearat | 0,7 | 0 | 1,8 | 1 | 1 |
| Carbopol 981 | 0,5 | 0,02 | 0,02 | 0 | 0 |
| Acrylic Acid/VP Crosspolymer | 0 | 0 | 0 | 0,5 | 0,75 |
| Trinatrium EDTA aq. | 0 | 1 | 1 | | 1 |
| Ethylhexylmethoxycinnamat + BHT | 0 | 0 | 0 | 2 | 0 |
| Butyl Methoxydibenzoylmethane | 0 | 0 | 0 | 0 | 2 |
| Phenylbenzimidazolesulfonsäure | 0 | 0 | 0 | 0 | 2 |
| Ethylhexylsalicylat | 0 | 0 | 0 | 0 | 2 |
| Titandioxid + Trimethoxycaprylylsilan | 0 | 0 | 0 | 0,3600 | 0 |
| Octocrylen | 0 | 0 | 0 | 0 | 2 |
| Parfum | 0,10 | 0,35 | 0,35 | 0 | 0,20 |
| Wasser | ad 100 | ad 100 | ad 100 | ad 100 | ad 100 |

### Hydrodispersionsgele

| **Beispiel Nr.** | **6** | **7** | **8** | **9** | **10** |
|---|---|---|---|---|---|
| Silikonöl, cyclisch | 8 | 10 | --- | 3 | --- |
| Silikonöl, linear | --- | --- | --- | --- | 3 |
| Dimethiconol | 1 | 2 | 3 | --- | 3 |
| Ethanol | 1,0 | 5,0 | 7,5 | 1,5 | 3,0 |
| Natriumpolyacrylat | 0,2 | 0,3 | 0,3 | 0,4 | 0,10 |
| Methylpropandiol | 2 | 3 | 4 | 5 | --- |
| Glycerin | 9 | 15 | 5 | 7,5 | 25 |
| Carbomer | 0,2 | 0,3 | 0,2 | 0,4 | 0,15 |
| Acrylate/C10-30Alkylacrylat Crosspolymer | 0,2 | 0,15 | 0,3 | 0,4 | 0,10 |
| Carrageenan (Chondrus Crispus) | --- | --- | --- | --- | 2 |
| Hamamelis Extrakt | 0,1 | 0,2 | 0,3 | 0,4 | --- |
| Parfum | q.s. | q.s. | q.s. | q.s. | q.s. |
| Octanohydroxamsäure | 0,25 | 0,2 | 0,3 | 0,1 | 0,2 |
| Glycerylcaprylat | 0,5 | 0 | 0,3 | 0 | 0,5 |
| Ethylhexylglycerin | 0 | 0,3 | 0 | 0,5 | 0,5 |
| Wasser | ad 100 | ad 100 | ad 100 | ad 100 | ad 100 |

| **O/W Emulsion** | **11** | **12** | **13** | **14** | **15** |
|---|---|---|---|---|---|
| Pottasium Cetyl Phosphate | 0 | 1,0 | 0 | 0 | 0,5 |
| Sodium Stearyl Glutamate | 1,0 | | 1,0 | 0 | 0 |
| Polyglyceryl-3 Methylglycose Distearat | 0 | 0 | 0 | 2,0 | 0 |
| Cetearyl Alcohol Sodium Cetearyl Sulfate | 0 | 0 | 0 | 0 | 0 |
| Glyceryl Stearat SE | 0 | 0 | 0,60 | 0 | 0,6 |
| Polyepsilon-Lysine (ε-Polylysin) | 2 | 1 | 0,5 | 0,25 | 4 |
| Stearylalkohol | 2 | 1,5 | 0 | 0 | 0 |
| Acrylates/C₁₀₋₃₀ Alkyl Acrylat Crosspolymer | 0 | 0,2 | 0 | 0 | 0,1 |
| Xanthan Gum | 0,4 | 0 | 0,2 | 0,2 | 0,3 |
| C₁₂₋₁₅ Alkyl Benzoat | 0 | 3 | 0 | 0 | 5 |
| Dicaprylyl Carbonat | 0 | 2 | 0 | 0 | 0 |
| Myristylmyristat | 0 | 0 | 2 | 0 | 1 |
| Butylenglycol Dicaprylat/Dicaprat | 0 | 0 | 3 | 0 | 3 |
| Propylheptyl Caprylat | 5 | 0 | 5 | 2 | |
| Dicaprylyl Ether | 0 | 0 | 0 | 0 | 2 |
| Cyclopentasilxonan | 0 | 5 | 0 | 0 | 10 |
| MT Propylsilsesquioxan Wachsharz mit M= Si (C 30+)(CH₃)₂ | 0 | 3 | 0 | 0 | 1,5 |
| Dimethicone | 6 | 0 | 0 | 5 | 0 |
| Dimethiconol | 0 | 5 | 0 | 0 | 0 |
| Glycerin | 3 | 0 | 12 | 10 | 5 |
| Bis-Vinyl Dimethicon / PPG-20 Crosspolymer | 0 | 4 | 1 | 0 | 0 |
| 2-(4'-Diethylamino-2'-hydoxybenzoyl)-benzoesäurehexylester | 3 | 5 | 1 | 0,5 | 2 |
| Octocrylen | 0 | 0 | 5 | 0 | 0 |
| Titandioxid | 0 | 0,5 | 1 | 0 | 0 |
| Phenylbenzimidazol Sulfonsäure | 0 | 0 | | 4 | 2 |
| Octylsalicylat | 0 | 0 | 5 | | |
| Polysilicon-15 | 0 | 0 | 0 | 0 | 2 |
| Ethylhexylmethoxycinnamat | 0 | 10 | 0 | 0 | 0 |
| Methylen Bis-Benzotriazolyl Tetramethylbutylphenol | 3 | 0 | 0 | 2 | 0 |
| Ethylhexyltriazon | 3 | 2 | 0 | 0 | 3 |
| Tris-Triphenyl Triazine | 0 | 0 | 2 | 0 | 0 |
| Bis-Ethylhexylphenol Methoxyphenyl Triazin | 0 | 2 | 0 | 0 | 0 |
| PVP/Hexadecen Copolymer | 0 | 0,5 | 0,1 | 0 | 0 |
| Vitamin E Acetat | 0,2 | 0,2 | 0,2 | 0,3 | 0,1 |
| Na₂H₂EDTA | 0,1 | 0,1 | 0 | 0 | 0,2 |
| Parfüm | 0,2 | 0,3 | 0,3 | 0,4 | 0,25 |
| Octanohydroxamsäure | 0 | 0 | 0,1 | 0,3 | 0,25 |
| Acetohydroxamsäure | 0,2 | 0,2 | 0 | 0 | 0 |
| Glycerylcaprylat | 0,5 | 0 | 0,3 | 0 | 0,5 |
| Ethylhexylglycerin | 0 | 0,3 | 0 | 0,5 | 0,5 |
| Natriumhydroxid | q.s. | q.s. | q.s. | q.s. | q.s. |
| Wasser | ad 100,0 | ad 100,0 | ad 100,0 | ad 100,0 | ad 100,0 |

### Wirknachweis:

Das Bakterium *S.aureus (ATCC 12000)* wurde aus einem nach EN 12353 angelegten Cryoröhrchen auf einer entsprechenden Agarplatte (TSA-Agar) ausgestrichen und bei 37°C 24 Stunden bebrütet. Von den gewachsenen Bakterien wurde eine zweite Passage angelegt, die in der Wachstumskurve eingesetzt wurde.

Eine Impföse der Bakterien wurde zu 10mL Verdünnungsmittel und 5g Glasperlen mit 4mm Durchmesser gegeben und 3 Minuten gevortext. Anschließend wurde diese Bakteriensuspension auf eine OD von 0,08 eingestellt, um eine Keimzahl von ungefähr 5*10⁷ zu gewährleisten. Diese Bakteriensuspension wurde anschließend für den Test eingesetzt.

Die Wachstumskurve wurde mittels TECAN-Pipettierroboter durchgeführt. Hierzu wurden 500µL der zu testenden Wirkstofflösungen und 500 µl der Bakteriensuspension in Eppendorfgefäßen ohne Deckel mit aufgeschweißter Folie im Cooling-Rack vorgelegt. Nach 1, 6 und 24 Stunden bei 20°C wurden aus den Eppendorfgefäßen jeweils 100µL entnommen und zu 800µL Neutralisationsmittel und 100µl Wasser pipettiert (10⁻¹-Verdünnung). Aus der 10⁻¹-Verdünnung wurde eine 10⁻³-Verdünnung hergestellt (990µl Verdünnungsmittel und 10µl aus der 10⁻¹-Verdünnung). Diese Verdünnungen wurden mittels Spiralplatter auf Agarplatten ausplattiert.

Eine Ausnahme stellt die Kontrolle da, die nur zu Beginn abgenommen wurde. Sie wurde nochmal 1:100 verdünnt (10⁻⁵-Verdünnung). Bei den Kontrollen wurden die 10⁻³ und 10⁻⁵ - Verdünnungen auf Agarplatten gebracht. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

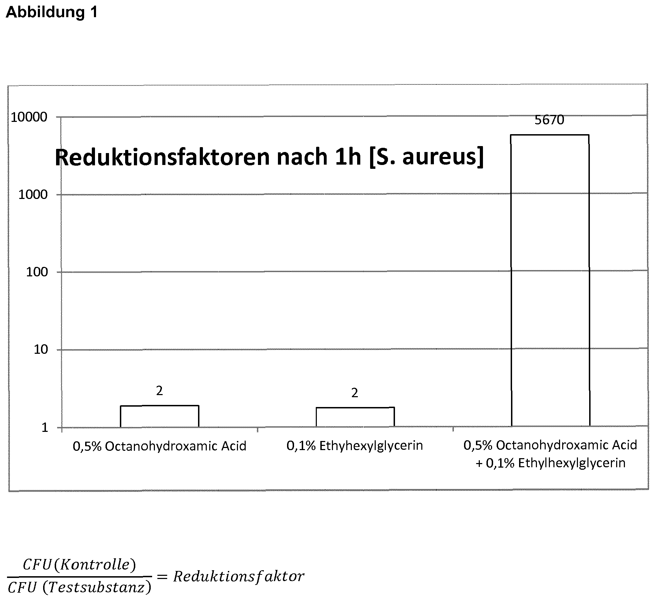

**Siehe Abbildung 1**

## Patentansprüche

1. Dermatologische Zubereitung zur Verwendung als antibakterielles, antimykotisches oder antivirales Mittel auf menschlicher Haut und/oder Haaren, bei der Behandlung eines dermatologischen Zustands, wobei die dermatologische Zubereitung eine Wirkstoffkombination umfasst, wobei die Wirkstoffkombination umfasst
a) eine oder mehrere physiologisch unbedenkliche Hydroxamsäuren, und
b) einen oder mehrere Alkylether des Glycerins der allgemeinen Strukturformel 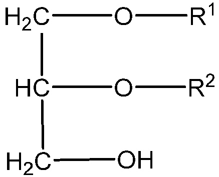 mit R¹ und R² unabhängig voneinander verzweigte oder unverzweigte Alkylreste mit 1 - 18 Kohlenstoffatomen,
wobei der eine oder mehrere Alkylether des Glycerins in einer Konzentration von 0,001 bis 5,0 Gew.-% der Zubereitung vorliegen.

2. Zubereitung zur Verwendung nach Anspruch 1, wobei das Gewichtsverhältnis der einen oder mehrerer physiologisch unbedenklichen Hydroxamsäuren zu dem der einen oder mehrerer Alkylether des Glycerins 1/5 bis 5/1 ist.

3. Zubereitung zur Verwendung nach einem der Ansprüche 1-2, wobei die eine oder mehrere physiologisch unbedenkliche Hydroxamsäuren in einer Konzentration von 0,001 bis 5,0 Gew.-% der Zubereitung vorhanden ist.

4. Zubereitung zur Verwendung nach einem der vorhergehenden Ansprüche 1 bis 3, wobei die eine oder mehrere physiologisch unbedenkliche Hydroxamsäuren Octanohydroxamsäure enthält.

5. Zubereitung zur Verwendung nach einem der Ansprüche 1-4, wobei die dermatologische Zubereitung ferner einen geeigneten dermatologischen Träger umfasst.

6. Zubereitung zur Verwendung nach einem der Ansprüche 1-5, wobei die dermatologische Zubereitung mindestens eine davon: eine Wasser-in-Öl-Emulsion, eine Öl-in-Wasser-Emulsion, eine Wasser-in-Öl-in-Wasser-Emulsion, ein Gel, eine Hydrodispersion, ein fester Stick oder ein Aerosol ist.

7. Zubereitung zur Verwendung nach einem der Ansprüche 1 bis 6, wobei die dermatologische Zubereitung gegen mindestens eines Mykobonten, milden Formen von Akne oder Propionisbacterium aknes, Streptokokken, Staphylococcus aureus, Pityrospori oder seborrhoische Dermatitis nützlich ist.

8. Zubereitung zur Verwendung nach einem der Ansprüche 1-7, wobei die dermatologische Zubereitung ein Deodorant ist.

9. Zubereitung zur Verwendung nach einem der Ansprüche 1-8, wobei die dermatologische Zubereitung ein Shampoo ist.

10. Zubereitung zur Verwendung nach einem der Ansprüche 1-9, wobei R¹ und R² jeweils unabhängig voneinander verzweigte Alkylreste mit 4 - 14 Kohlenstoffatomen sind.

11. Zubereitung zur Verwendung nach einem der Ansprüche 1-10, wobei das Gewichtsverhältnis der einen oder mehrerer physiologisch unbedenklichen Hydroxamsäuren zu dem der einen oder mehrerer Alkylether des Glycerins 1/2 bis 2/1 ist.

12. Zubereitung zur Verwendung nach einem der Ansprüche 1-11, wobei die eine oder mehrere physiologisch unbedenkliche Hydroxamsäuren in einer Konzentration von 0,01 bis 2,0 Gew.-% der Zubereitung vorliegt.

## Claims

1. Dermatological preparation for use as an antibacterial, antimycotic or antiviral agent on human skin and/or hair, in the treatment of a dermatological condition, wherein the dermatological preparation comprises an active substance combination, wherein the active substance combination comprises
a) one or more physiologically acceptable hydroxamic acids, and
b) one or more alkyl ethers of glycerol of the general structural formula 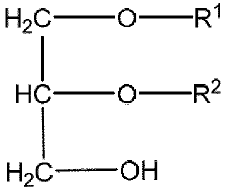 where R¹ and R² independently of one another are branched or unbranched alkyl radicals having 1-18 carbon atoms,
wherein the one or more alkyl ethers of glycerol are present in a concentration of from 0.001 to 5.0 wt.% of the preparation.

2. Preparation for use according to claim 1, wherein the weight ratio of the one or more physiologically acceptable hydroxamic acids to the one or more alkyl ethers of glycerol is 1/5 to 5/1.

3. Preparation for use according to one of claims 1-2, wherein the one or more physiologically acceptable hydroxamic acids is present in a concentration of from 0.001 to 5.0 wt.% of the preparation.

4. Preparation for use according to one of the preceding claims 1 to 3, wherein the one or more physiologically acceptable hydroxamic acids contains octanohydroxamic acid.

5. Preparation for use according to one of claims 1-4, wherein the dermatological preparation also comprises a suitable dermatological carrier.

6. Preparation for use according to one of claims 1-5, wherein the dermatological preparation is at least one from among: a water-in-oil emulsion, an oil-in-water emulsion, a water-in-oil-in-water emulsion, a gel, a hydrodispersion, a solid stick or an aerosol.

7. Preparation for use according to one of claims 1-6, wherein the dermatological preparation is effective against at least one of mycobionts, mild forms of acne or Propionibacterium acnes, streptococci, Staphylococcus aureus, Pityrosporum or seborrhoeic dermatitis.

8. Preparation for use according to one of claims 1-7, wherein the dermatological preparation is a deodorant.

9. Preparation for use according to one of claims 1-8, wherein the dermatological preparation is a shampoo.

10. Preparation for use according to one of claims 1-9, wherein R¹ and R² are each independently of one another branched alkyl radicals having 4 - 14 carbon atoms.

11. Preparation for use according to one of claims 1-10, wherein the weight ratio of the one or more physiologically acceptable hydroxamic acids to the one or more alkyl ethers of glycerol is 1/2 to 2/1.

12. Preparation for use according to one of claims 1-11, wherein the one or more physiologically acceptable hydroxamic acids is present in a concentration of from 0.01 to 2.0 wt.% of the preparation.

## Revendications

1. Préparation dermatologique destinée à une utilisation comme agent antibactérien, antimycotique ou antiviral sur la peau et/ou les cheveux humains, dans le cadre du traitement d'une affection dermatologique, ladite préparation dermatologique renfermant une combinaison de principes actifs, ladite combinaison de principes actifs renfermant
a) un ou plusieurs acides hydroxamiques physiologiquement acceptables, et
b) un ou plusieurs éthers alkyliques de glycérine correspondant à la formule développée 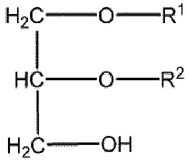 dans laquelle R¹ et R² représentent indépendamment des restes alkyle linéaires ou ramifiés comptant 1 à 18 atomes de carbone,
le ou les éthers alkyliques de glycérine étant présents dans une concentration de l'ordre de 0,001 à 5,0 % en poids de la préparation.

2. Préparation destinée à une utilisation selon la revendication 1, dans laquelle le rapport en poids du ou des acides hydroxamiques physiologiquement acceptables à celui du ou des éthers alkyliques de glycérine est de l'ordre de 1/5 à 5/1.

3. Préparation destinée à une utilisation selon l'une des revendications 1 et 2, dans laquelle le ou les acides hydroxamiques physiologiquement acceptables sont présents dans une concentration de l'ordre de 0,001 à 5,0 % en poids de la préparation.

4. Préparation destinée à une utilisation selon l'une des revendications précédentes 1 à 3, dans laquelle le ou les acides hydroxamiques physiologiquement acceptables comprennent l'acide octanohydroxamique.

5. Préparation destinée à une utilisation selon l'une des revendications 1 à 4, dans laquelle la préparation dermatologique renferme en outre un véhicule dermatologique approprié.

6. Préparation destinée à une utilisation selon l'une des revendications 1 à 5, dans laquelle la préparation dermatologique consiste en au moins une des formes suivantes : une émulsion eau dans huile, une émulsion huile dans eau, une émulsion eau dans huile dans eau, un gel, une hydrodispersion, un bâton solide ou un aérosol.

7. Préparation destinée à une utilisation selon l'une des revendications 1 à 6, dans laquelle la préparation dermatologique est utile contre au moins l'un des états suivants : mycobiontes, formes légères de l'acné ou de Propionibacterium acnes, streptocoques, Staphylococcus aureus, Pityrospori ou dermatite séborrhéique.

8. Préparation destinée à une utilisation selon l'une des revendications 1 à 7, dans laquelle la préparation dermatologique est un déodorant.

9. Préparation destinée à une utilisation selon l'une des revendications 1 à 8, dans laquelle la préparation dermatologique est un shampooing.

10. Préparation destinée à une utilisation selon l'une des revendications 1 à 9, dans laquelle R¹ et R² représentent, indépendamment l'un de l'autre, des restes alkyle ramifiés comptant 4 à 14 atomes de carbone.

11. Préparation destinée à une utilisation selon l'une des revendications 1 à 10, dans laquelle le rapport en poids du ou des acides hydroxamiques physiologiquement acceptables à celui du ou des éthers alkyliques de glycérine est de l'ordre de 1/2 à 2/1.

12. Préparation destinée à une utilisation selon l'une des revendications 1 à 11, dans laquelle le ou les acides hydroxamiques physiologiquement acceptables sont présents dans une concentration de 0,01 à 2,0 % en poids de la préparation.
